# EUROPÄISCHE PATENTSCHRIFT

(11) **EP 0 684 288 B1**
(45) Veröffentlichungstag und Bekanntmachung des Hinweises auf die Patenterteilung: **12.02.1997**
(21) Anmeldenummer: 95107162.0
(22) Anmeldetag: 11.05.1995
(51) Int. Cl.: C09B 43/00, C07D 209/62, C09B 29/085, C09B 29/095, C09B 35/215

(54) **Verfahren zur Herstellung von Aminoazofarbstoffen**
Process for the preparation of aminoazo dyes
Procédé pour la préparation de colorants aminoazoiques

(30) Priorität: 20.05.1994 US 246750
(43) Veröffentlichungstag der Anmeldung: 29.11.1995
(73) Patentinhaber: BASF Aktiengesellschaft, 67063 Ludwigshafen (DE)
(72) Erfinder: Bermes, Rudolf, Dr., D-67069 Ludwigshafen (DE); Keilhauer, Heinz, D-67134 Birkenheide (DE)

(56) Entgegenhaltungen:
- US-A- 2 139 325
- R.STROH 'Houben-Weyl, Methoden der Organischen Chemie' 1965 , GEORG THIEME VERLAG , STUTTGART 4. Auflage, Band 10/3, Stikstoffverbindungen-1, K.H.Schündehütte "Dyarylazoverbindungen" * Seite 242 *
- CHEMICAL ABSTRACTS, vol. 106, no. 6, 9.Februar 1987 Columbus, Ohio, US; abstract no. 34619f, R.LOEFFLER ET AL. '4-(4-aminophenylazo)benzenesulfonic acid' Seite 86;

## Beschreibung

Die vorliegende Erfindung betrifft ein neues Verfahren zur Herstellung von Aminoazofarbstoffen durch wäßrig-saure Hydrolyse der entsprechenden N-sulfomethylierten Aminoazofarbstoffe.

Aus Houben-Weyl "Methoden der Organischen Chemie", 4. Auflage, Band 10/3, Seite 242, 1965, ist bereits die Hydrolyse von N-sulfomethylierten Aminoazofarbstoffen bekannt.

Es hat sich jedoch gezeigt, daß die dort beschriebenen Verfahren Nachteile aufweisen. So ist z.B. die Hydrolysegeschwindigkeit relativ gering. Außerdem fallen die Aminoazofarbstoffe dabei in unbefriedigender Ausbeute und Reinheit an.

Aufgabe der vorliegenden Erfindung war es, ein neues Verfahren zur sauren Hydrolyse von N-sulfomethylierten Aminoazofarbstoffen bereitzustellen, bei dem die Zielprodukte in hoher Ausbeute und Reinheit anfallen. Zugleich sollte die Hydrolyse in kurzer Zeit ablaufen.

Es wurde nun gefunden, daß die Herstellung von Aminoazofarbstoffen der Formel I 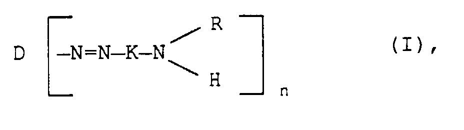 in der
- n: 1 oder 2,
- D,: wenn n 1 ist, gegebenenfalls substituiertes Phenyl oder gegebenenfalls substituiertes Naphthyl oder, wenn n 2 ist, gegebenenfalls substituiertes Stilbendiyl,
- K: gegebenenfalls substituiertes Phenylen oder gegebenenfalls substituiertes Naphthylen und
- R: Wasserstoff oder C₁-C₄-Alkyl bedeuten,
durch wäßrig-saure Hydrolyse von sulfomethylierten Aminoazofarbstoffen der Formel II 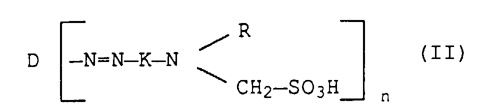 oder deren Salzen, worin n, D, K und R jeweils die obengenannte Bedeutung besitzen, vorteilhaft gelingt, wenn man die Hydrolyse in Gegenwart von Amidosulfonsäure, Harnstoff oder deren Gemischen vornimmt.

Zur Hydrolyse kann man die sulfomethylierten Aminoazofarbstoffe der Formel II entweder in Form der freien Säure oder in Form ihrer Salze anwenden.

Geeignete Salze sind z.B. die Alkali- oder Erdalkalisalze, wie die Lithium-, Natrium-, Kalium-, Magnesium- oder Calciumsalze, oder Ammoniumsalze.

Die Anwendung der Natrium- oder Kaliumsalze ist bevorzugt.

Alle in den obengenannten Formeln I und II auftretenden Alkylreste können sowohl geradkettig als auch verzweigt sein.

Wenn die Reste D und K substituiert sind, so weisen sie in der Regel 1 bis 4, vorzugsweise 1 bis 3, Substituenten auf.

Geeignete Substituenten sind z.B. C₁-C₄-Alkyl, C₁-C₄-Alkoxy, Hydroxy, Halogen, Nitro, Hydroxysulfonyl, Carboxyl, Cyano oder C₁-C₄-Alkanoylamino.

C₁-C₄-Alkylreste sind z.B. Methyl, Ethyl, Propyl, Isopropyl, Butyl, Isobutyl, sec-Butyl oder tert-Butyl.

C₁-C₄-Alkoxyreste sind z.B. Methoxy, Ethoxy, Propoxy, Isopropoxy, Butoxy, Isobutoxy, sec-Butoxy oder tert-Butoxy.

Halogen ist z.B. Fluor, Chlor oder Brom.

C₁-C₄-Alkanoylamino ist z.B. Formylamino, Acetylamino, Propionylamino, Butyrylamino oder Isobutyrylamino.

Bevorzugt ist eine Verfahrensweise zur Herstellung von Aminoazofarbstoffen der Formel I, in der n 1 bedeutet.

Weiterhin bevorzugt ist eine Verfahrensweise zur Herstellung von Aminoazofarbstoffen der Formel I, in der R Wasserstoff bedeutet.

Weiterhin bevorzugt ist eine Verfahrensweise zur Herstellung von Aminoazofarbstoffen der Formel I, in der D gegebenenfalls substituiertes Phenyl bedeutet.

Weiterhin bevorzugt ist eine Verfahrensweise zur Herstellung von Aminoazofarbstoffen der Formel I, in der K gegebenenfalls substituiertes Phenylen bedeutet.

Besonders bevorzugt ist eine Verfahrensweise zur Herstellung von Aminoazofarbstoffen der Formel I, in der D Phenyl, das gegebenenfalls durch C₁-C₄-Alkyl, Halogen, Nitro, Hydroxysulfonyl oder Carboxyl substituiert ist, bedeutet.

Weiterhin besonders bevorzugt ist eine Verfahrensweise zur Herstellung von Aminoazofarbstoffen der Formel I, in der K Phenylen, das gegebenenfalls durch C₁-C₄-Alkyl, C₁-C₄-Alkoxy oder Halogen substituiert ist, bedeutet.

Die Hydrolyse der sulfomethylierten Aminoazofarbstoffe der Formel II findet in wäßrig-saurem Medium statt. Geeignete wäßrig-saure Medien sind z.B. wäßrige Lösungen von anorganischen oder organischen Säuren, beispielsweise Halogenwasserstoffsäuren, wie Salzsäure oder Bromwasserstoffsäure, Schwefelsäure, Amidosulfonsäure, Phosphorsäure, Ameisensäure, Benzolsulfonsäure oder Toluolsulfonsäure.

Die Säurekonzentration im wäßrig-sauren Medium beträgt üblicherweise 0,5 bis 15 Gew.-%, vorzugsweise 1 bis 10 Gew.-%, jeweils bezogen auf das Gewicht des Reaktionsmediums.

Die Menge an wäßrig-saurem Reaktionsmedium ist nicht kritisch, jedoch sollte darauf geachtet werden, daß das Reaktionsgemisch rührbar bleibt.

Erfindungsgemäß wird das neue Verfahren in Gegenwart von Amidosulfonsäure, Harnstoff oder deren Gemischen durchgeführt. Je Moläquivalent sulfomethyliertem Aminoazofarbstoff der Formel II wendet man zweckmäßig 0,5 bis 5 mol, vorzugsweise 1 bis 2,5 mol und insbesondere 1 bis 2 mol dieser Reagentien an.

Die Verwendung von Amidosulfonsäure ist bevorzugt.

Amidosulfonsäure kann dabei gleichzeitig als Säurekomponente für das wäßrig-saure Medium und als Hydrolyseverbesserungsmittel dienen. Dabei sind die obengenannten Mengenverhältnisse zu berücksichtigen.

Das erfindungsgemäße Verfahren wird in der Regel bei einer Temperatur von 60 bis 120°C, vorzugsweise 70 bis 95°C, vorgenommen.

Im allgemeinen ist die Hydrolyse innerhalb von kurzer Zeit, üblicherweise 15 bis 60 Minuten, beendet.

Bevorzugt ist eine Verfahrensweise, bei der man nicht von zwischenisoliertem sulfomethylierten Aminoazofarbstoff der Formel II ausgeht, sondern vom Reaktionsgemisch, wie es bei dessen Synthese anfällt. Das heißt, man diazotiert zunächst ein Amin der Formel III

D[-NH₂]ₙ (III),

in der n und D jeweils die obengenannte Bedeutung besitzen, auf übliche Weise und kuppelt es mit einer sulfomethylierten Kupplungskomponente der Formel IV 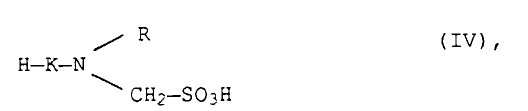 in der K und R jeweils die oben genannte Bedeutung besitzen. Das resultierende Reaktionsgemisch kann dann, wie oben dargelegt, direkt der sauren Hydrolyse unterworfen werden.

Das neue Verfahren wird zweckmäßig so durchgeführt, daß man eine wäßrig-saure Lösung oder Suspension des sulfomethylierten Aminoazofarbstoffs der Formel II mit Amidosulfonsäure, Harnstoff oder deren Gemisch versetzt und unter Rühren auf die obengenannte Temperatur erwärmt. Nach Beendigung der Hydrolyse, die sich beispielsweise chromatographisch verfolgen läßt, kühlt man das Reaktionsgemisch ab und isoliert den üblicherweise in fester Form vorliegenden Aminoazofarbstoff der Formel I, beispielsweise durch Absaugen, Waschen mit Wasser und Trocknen.

Mittels des neuen Verfahrens gelingt es, die Aminoazofarbstoffe der Formel I in hoher Ausbeute herzustellen. Die Farbstoffe fallen dabei in sehr guter Reinheit an.

Die mittels des erfindungsgemäßen Verfahrens erhältlichen Verbindungen I der Formel I besitzen einerseits Farbstoffcharakter und sind andererseits wertvolle Zwischenprodukte für die Herstellung weiter substituierter Farbstoffe, z.B. Polyazofarbstoffe.

Die folgenden Beispiele sollen die Erfindung näher erläutern.

### Beispiel 1

173 g Sulfanilsäure wurden in 1000 ml Eiswasser in Gegenwart von 60 g konz. Schwefelsäure mit 69 g Natriumnitrit auf übliche Weise bei einem pH-Wert von 1,3 diazotiert. Die Suspension wurde unter Rühren mit 100 g Natriumhydrogencarbonat abgestumpft und mit 220 g Anilinomethansulfonsäure-Natriumsalz versetzt. Die Kupplung war über Nacht beendet. Der pH-Wert des Gemisches betrug 5,7. Man fügte nun 100 g Amidosulfonsäure und 100 g konz. Schwefelsäure, die mit ein wenig Eis verdünnt wurde, hinzu, erwärmte und rührte 15 min bei 95°C. Danach kühlte man den Ansatz auf Raumtemperatur ab und saugte den Niederschlag ab. Der mit 3000 ml Wasser gewaschene Filterkuchen wurde bei 80°C getrocknet und ergab 256 g 4'-Aminoazobenzol-4-sulfonsäure mit einem Reingehalt von 98,6 %.

### Beispiel 2

Man arbeitete analog Beispiel 1, ersetzte jedoch die Amidosulfonsäure durch 100 g konz. Schwefelsäure. Man erhielt nach zweistündiger Hydrolyse bei 95°C nur 220 g des Farbstoffs mit einem Reingehalt von lediglich 88,0 %.

### Beispiel 3

Man führte die Kupplungsreaktion analog Beispiel 1 durch, jedoch verwendete man jeweils nur die 0,6-fache Menge der Reaktionspartner. Dann teilte man das resultierende Kupplungsgemisch in 3 gleichgroße Teile auf.
a) Der erste Teil wurde mit 20 g Amidosulfonsäure und 20 g konz. Schwefelsäure versetzt und bei 95°C zur Abspaltung des Sulfomethylrestes gebracht. Man erhielt 44,2 g 4'-Aminoazobenzol-4-sulfonsäure mit einem Reingehalt von 97,6 %.
b) Beim zweiten Teil verwendete man zur Abspaltung 20 g Harnstoff und 30 Teile konz. Schwefelsäure und erhielt 41,1 g des Farbstoffs mit einem Reingehalt von 98,7 %.
c) Beim letzten Drittel verwendete man lediglich 30 g konz. Schwefelsäure. Die Ausbeute betrug 37,2 g Farbstoff bei einem Reingehalt von 91,9 %.

### Beispiel 4

Man führte die Kupplungsreaktion analog Beispiel 1 durch, jedoch verwendete man jeweils nur die 0,5-fache Menge der Reaktionspartner. Das resultierende Kupplungsgemisch wurde mit 200 g Ameisensäure und 50 g Amidosulfonsäure versetzt und 1 h lang bei 95°C gerührt. Nach der Aufarbeitung erhielt man 101,2 g 4'-Aminoazobenzol-4-sulfonsäure mit einem Reingehalt von 96,9 %.

### Beispiel 5

Man verfuhr analog Beispiel 4, verwendete jedoch nur 200 g Ameisensäure bei der Abspaltung des Sulfomethylrestes. Die Ausbeute betrug nur 78,8 g Farbstoff mit einem Reingehalt von nur 79,8 %.

### Beispiel 6

Man verfuhr analog Beispiel 4, verwendete jedoch anstelle von Amidosulfonsäure die gleiche Menge an Harnstoff. Die Ausbeute betrug 103,8 g Farbstoff mit einem Reingehalt von 97,3 %.

### Beispiel 7

Man verfuhr analog Beispiel 4, verwendete jedoch anstelle von Ameisensäure die gleiche Menge Benzolsulfonsäure bei der Abspaltung des Sulfomethylrestes. Man erhielt 104,8 g Farbstoff mit einem Reingehalt von 96,0 %.

### Beispiel 8

Man verfuhr analog Beispiel 7, unterließ jedoch den Zusatz von Amidosulfonsäure. Die Ausbeute betrug 96,0 g Farbstoff mit einem Reingehalt von 93,0 %.

### Beispiel 9

Man verfuhr analog Beispiel 1, verwendete jedoch anstelle der Sulfanilsäure die äquivalente Menge an 2-Aminotoluol-5-sulfonsäure. Man erhielt 250 g 4'-Amino-2-methylazobenzol-4-sulfonsäure mit einem Reingehalt von 98 %.

### Beispiel 10

Man verfuhr analog Beispiel 9 verwendete jedoch anstelle von Anilinomethansulfonsäure-Natriumsalz die äquivalente Menge o-Toluidinomethansulfonsäure-Natriumsalz und erhöhte, damit der bei der Hydrolyse entstandene Kristallbrei rührbar blieb, die Wassermenge soweit, daß der Ansatz ein Volumen von etwa 10 l einnahm. Man erhielt 262 g 4'-Amino-3',2-dimethylazobenzol-4-sulfonsäure mit einem Reingehalt von 96,9 %.

### Beispiel 11

137 g 3-Aminobenzoesäure und 90 g 50 gew-%ige Natronlauge wurden in 1000 ml Wasser gelöst und mit einer Lösung von 70 g Natriumnitrit in 300 ml Wasser vermischt. Man rührte 500 g Eis unter und ließ diese Lösung bei 0 bis 5°C unter Außenkühlung innerhalb von 30 min in eine gerührte Mischung aus 135 g konz. Schwefelsäure und 500 g Eis einfließen. Nach 2 h zerstörte man den Nitritüberschuß mit wenig Amidosulfonsäure, filtrierte die Diazoniumsalzlösung und gab sie zu einer mit 220 g Natriumhydrogencarbonat versetzten Suspension von 240 g o-Anisidinomethansulfonsäure-Natriumsalz in 500 ml Wasser. Man rührte über Nacht weiter, fügte 100 g Amidosulfonsäure und 100 g konz. Schwefelsäure zum Reaktionsgemisch und rührte 1 h bei 95°C. Die Aufarbeitung lieferte 254 g 4'-Amino-3'-methoxyazobenzol-3-carbonsäure mit einem Reingehalt von 97,2 %.

### Beispiel 12

Man verfuhr analog Beispiel 1, verwendete jedoch anstelle der Sulfanilsäure die äquivalente Menge an 4-Amino-2,5-dichlorbenzolsulfonsäure. Man erhielt 310 g 4'-Amino-2,5-dichlorazobenzol-4-sulfonsäure mit einem Reingehalt von 97,7 %.

### Beispiel 13

Man verfuhr analog Beispiel 1, jedoch kuppelte man die diazotierte Sulfanilsäure mit der äquivalenten Menge N-(Hydroxysulfonylmethyl)-3-chloranilin und hydrolysierte anschließend mit 250 g Amidosulfonsäure bei 95°C. Man erhielt nach Absaugen, Waschen und Trocknen 209 g 2'-Chlor-4'-aminoazobenzol-4-sulfonsäure mit einem Reingehalt von 99,3 %.

### Beispiel 14

Man verfuhr analog Beispiel 1, verwendete jedoch anstelle der Sulfanilsäure die äquivalente Menge an 2-Aminonaphthalin-6-sulfonsäure. Man erhielt 298 g 2-(4'-Aminophenylazo)naphthalin-6-sulfonsäure mit einem Reingehalt von 96,8 %.

### Beispiel 15

a) In 350 ml Wasser löste man bei Raumtemperatur nacheinander 16 g 50 gew.-%ige Natronlauge, 37,4 g 4,4'-Diaminostilben-2,2'-disulfonsäure und 14 g Natriumnitrit. Die resultierende Lösung ließ man unter Rühren in eine Mischung aus 100 ml Wasser, 200 g Eis und 208 g 5 N Salzsäure einlaufen. Ein geringer Überschuß an salpetriger Säure wurde nach einstündigem Rühren bei 5 bis 10°C durch den Zusatz von 0,2 g Amidosulfonsäure zerstört.
b) In einem zweiten Reaktionsgefäß wurde tags zuvor bei 60°C aus 42 ml Wasser, 21 g 30 gew.-%iger wäßriger Formaldehydlösung und 70,6 g 30 gew.-%iger wäßriger Natriumhydrogensulfitlösung eine Lösung hergestellt, die man zu einer 45°C warmen Lösung von 47,9 g 1-Hydroxy-7-aminonaphthalin-3-sulfonsäure -3-sulfonsäure und 15,2 g 60 gew.-%iger Natronlauge in 240 ml mit einem pH-Wert von 6 einlaufen ließ und über Nacht bei Raumtemperatur rührte.
c) Das unter a) beschriebene Reaktionsgemisch setzte man unter Rühren der unter b) beschriebenen Mischung zu, wobei der pH-Wert durch Zugabe von 24 g Natriumcarbonat zwischen 7,5 und 8,5 gehalten wurde. Nach beendeter Kupplungsreaktion verdünnte man das resultierende Reaktionsgemisch auf ein Volumen von 2 1, säuerte mit 200 g Amidosulfonsäure an, erwärmte auf 95 bis 100°C und rührte 30 min bei dieser Temperatur nach. Nach dem Abkühlen wurde der enstandene Farbstoff abgesaugt und getrocknet. Man erhielt 169 g des salzhaltigen, aber isomerenfreien Farbstoffs 4,4'-Bis(7-amino-1-hydroxy-3-hydroxysulfonylnaphth-2-ylazo)stilben-2,2'-disulfonsäure.

## Patentansprüche

1. Verfahren zur Herstellung von Aminoazofarbstoffen der Formel I 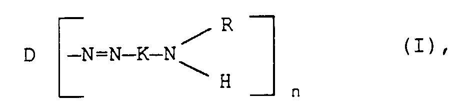 in der
n 1 oder 2,
D, wenn n 1 ist, gegebenenfalls substituiertes Phenyl oder gegebenenfalls substituiertes Naphthyl oder, wenn n 2 ist, gegebenenfalls substituiertes Stilbendiyl,
K gegenenfalls substituiertes Phenylen oder gegebenenfalls substituiertes Naphthylen und
R Wasserstoff oder C₁-C₄-Alkyl bedeuten,
durch wäßrig-saure Hydrolyse von sulfomethylierten Aminoazofarbstoffen der Formel II 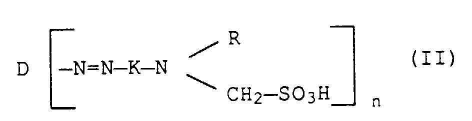 oder deren Salzen, n, worin D, K und R jeweils die obengenannte Bedeutung besitzen, dadurch gekennzeichnet, daß man die Hydrolyse in Gegenwart von Amidosulfonsäure, Harnstoff oder deren Gemischen vornimmt.

2. Verfahren nach Anspruch 1 , dadurch gekennzeichnet, daß n 1 bedeutet.

3. Verfahren nach Anspruch 1, dadurch gekennzeichnet, daß R Wasserstoff bedeutet.

4. Verfahren nach Anspruch 1, dadurch gekennzeichnet, daß D gegebenenfalls substituiertes Phenyl bedeutet.

5. Verfahren nach Anspruch 1, dadurch gekennzeichnet, daß K gegebenenfalls substituiertes Phenylen bedeutet.

6. Verfahren nach Anspruch 1, dadurch gekennzeichnet, daß man die Hydrolyse bei einer Temperatur von 60 bis 120°C vornimmt.

7. Verfahren nach Anspruch 1, dadurch gekennzeichnet, daß man je mol sulfomethyliertem Aminoazofarbstoff der Formel II 0,5 bis 5 mol Amidosulfonsäure, Harnstoff oder deren Gemische anwendet.

8. Verfahren nach Anspruch 1, dadurch gekennzeichnet, daß man die Hydrolyse in Gegenwart von Amidosulfonsäure vornimmt.

## Claims

1. A process for preparing aminoazo dyes of the formula I 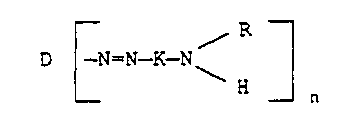 where
n is 1 or 2
D when n is 1, is substituted or unsubstituted phenyl or substituted or unsubstituted naphthyl or, when n is 2, is substituted or unsubstituted stilbenediyl,
K is substituted or unsubstituted phenylene or substituted or unsubstituted naphthylene, and
R is hydrogen or C₁-C₄-alkyl,
by aqueous acidic hydrolysis of sulfomethylated aminoazo dyes of the formula II 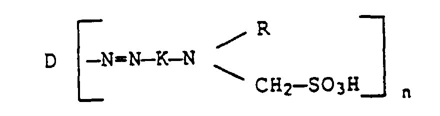 where n, D, K and R are each as defined above, or salts thereof, which comprises hydrolyzing in the presence of amidosulfuric acid, urea or mixtures thereof.

2. A process as claimed in claim 1, wherein n is 1.

3. A process as claimed in claim 1, wherein R is hydrogen.

4. A process as claimed in claim 1, wherein D is substituted or unsubstituted phenyl.

5. A process as claimed in claim 1, wherein K is substituted or unsubstituted phenylene.

6. A process as claimed in claim 1, wherein the hydrolysis is carried out at from 60 to 120°C.

7. A process as claimed in claim 1, wherein from 0.5 to 5 mol of amidosulfuric acid, urea or mixtures thereof are used per mole of sulfomethylated aminoazo dye of the formula II.

8. A process as claimed in claim 1, wherein the hydrolysis is carried out in the presence of amidosulfuric acid.

## Revendications

1. Procédé de préparation de colorants aminoazoïques de formule I 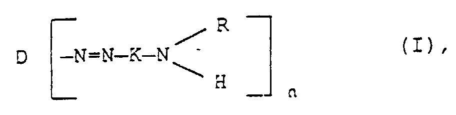 dans laquelle
n est mis pour 1 ou 2,
D représente un radical phényle éventuellement substitué ou un reste naphtyle éventuellement substitué lorsque n est mis pour 1 et un reste stilbènediyle éventuellement substitué lorsque n est mis pour 2,
K représente un reste phénylène éventuellement substitué ou naphtylène éventuellement substitué,
R représente un atome d'hydrogène ou un reste alkyle en C₁-C₄,
par hydrolyse hydro-acide de colorants aminoazoiques sulfométhylés de formule II 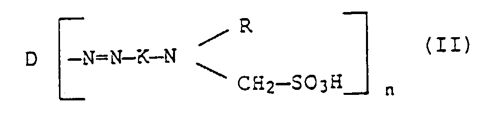 ou de leurs sels, n, D, K et R ayant chacun la signification donnée ci-dessus, caractérisé en ce que l'on procède à l'hydrolyse en présence d'acide amidosulfonique, d'urée ou de mélanges de ceux-ci.

2. Procédé selon la revendication 1, caractérise en ce que n est mis pour 1.

3. Procédé selon la revendication 1, caractérisé en ce que R représente un atome d'hydrogène.

4. Procédé selon la revendication 1, caractérisé en ce que D représente un reste phényle éventuellement substitué.

5. Procédé selon la revendication 1, caractérisé en ce que K représente un reste phénylène éventuellement substitué.

6. Procédé selon la revendication 1, caractérise en ce que l'on procède à l'hydrolyse à une température de 60 à 120°C.

7. Procédé selon la revendication 1, caractérisé en ce que l'on utilise 0,5 à 5 moles d'acide amidosulfonique, d'urée ou de leurs mélanges par mole de colorant aminoazoïque sulfométhylé de formule II.

8. Procédé selon la revendication 1, caractérisé en ce que l'on procède à l'hydrolyse en présence d'acide amidosulfonique.
